# EUROPEAN PATENT SPECIFICATION

(11) **EP 0 760 692 B1**
(45) Date of publication and mention of the grant of the patent: **22.12.2004**
(21) Application number: 94918165.5
(22) Date of filing: 26.05.1994
(51) Int. Cl.: A61M 5/178, A61M 39/06

(54) **SELF-SEALING VALVE DEVICE FOR ANGIOGRAPHIC CATHETERS**
SELBSTSCHLIESSENDE VENTILVORRICHTUNG FÜR ANGIOGRAFISCHEN KATHETER
DISPOSITIF A SOUPAPE A FERMETURE AUTOMATIQUE POUR CATHETERS ANGIOGRAPHIQUES

(43) Date of publication of application: 12.03.1997
(73) Proprietor: NATHAN PALESTRANT, as Trustee for THE AUBREY AND FAYE PALESTRANT IRREVOCABLE TRUST, Scottsdale, AZ 85253 (US)
(72) Inventor: PALESTRANT, Aubrey, M., Paradise Valley, AZ 85253 (US)
(74) Representative: Wächtershäuser, Günter, Prof. Dr.
(86) International application number: PCT/US1994/006073
(87) International publication number: WO 1995/032748

(56) References cited:
- US-A- 5 156 596

## Description

The present invention relates generally to angiographic catheters used to access a blood vessel, and more particularly, to devices for sealing the external end of an angiographic catheter or needle following insertion of such devices into the vascular system.

### Background Art

Catheters and needles are inserted into the vascular system for many reasons including, diagnosis, therapy or to draw blood samples. During an angiographic procedure, a catheter is placed into the vascular system commonly using a Seldinger technique. This technique consists of placing a needle percutaneously into a blood vessel of the vascular system, and then threading a flexible guidewire through the lumen of the needle into the blood vessel. The guidewire is left in place within the blood vessel, and the needle is thereafter removed. The distal tip of the catheter is then threaded over the external end of the guidewire and advanced therealong and positioned within the vascular system in accordance with the prior placement of the guidewire; the guidewire is thereafter removed. Diagnostic fluids may then be injected into the blood vessel to diagnose or treat various vascular conditions.

The steps described above must often be repeated during a procedure in order to manipulate the catheter, i.e., to move the catheter into various branches of the vascular system. To perform such manipulation, the guidewire is reinserted into the catheter, and the guidewire and catheter are then manipulated to the new position.

The angiographic catheter includes a fluid-communicating lumen extending between the distal end and proximal end thereof. Upon introduction of the distal end of the catheter into the blood vessel, blood pressure within the body forces blood into the catheter lumen. Some blood may escape from the proximal end of the catheter even before the guidewire is removed by flowing through the annulus between the guidewire and the inner walls of the catheter. Once the guidewire is withdrawn from the catheter, the outward flow of blood through the lumen of the catheter is essentially unimpeded.

Physicians prefer to minimize the amount of blood leakage from angiographic catheters for two compelling reasons. First, it is important to prevent blood loss from the patient to avoid the need for blood transfusions. Secondly, blood may contain highly infectious material such as hepatitis or AIDS to which medical personnel are exposed, and it is therefore beneficial to medical personnel to limit blood loss.

In order to prevent blood from freely draining out of the angiographic catheter, a first port of a stopcock is commonly secured by means of a luer lock connection to the proximal end of the catheter external to the patient. Such stopcocks are well known and include a manually rotatable handle or lever operated by the thumb and forefinger for selectively opening or sealing the proximal end of the catheter. Such stopcocks typically include a second port, also provided with a luer lock connection fitting, in fluid communication with the first port thereof when the stopcock is open. In order to draw blood from the patient into a syringe, or to inject a substance into the blood vessel through the catheter, the stopcock must be turned by hand into the opened position. When the catheter is not being used to perform aspiration or infusion, it is standard medical practice to inject heparinized saline through the catheter while closing the stopcock. This anticoagulant solution flushes any blood from the catheter and thereafter prevents blood from entering the catheter and clotting inside.

As described above, during vascular procedures involving catheters, it is common to pass a guidewire into the proximal end of the catheter to help guide the catheter tip into a selected blood vessel. If a stopcock has been secured to the proximal end of the catheter, then the stopcock must first be opened so that the distal tip of the guidewire may be passed through the opened stopcock into the catheter. When the stopcock is opened, the heparinized saline that was previously injected into the catheter is flushed out by the blood. The guidewire is passed into the catheter which now contains stagnant blood which can form blood clots between the guidewire and the inner wall of the catheter. Moreover, during guidewire manipulations, the stopcock must be left open to permit passage of the guidewire; accordingly, blood can flow between the guidewire and inner lumen of the catheter, and often drips out at the second port of the stopcock. This slow flow of escaping blood may form clots within the catheter; these blood clots can be forced back into the vascular system when the guidewire is advanced or when subsequent injections are made through the catheter. Introduction of such blood clots can result in blocked blood vessels; if these blood vessels are critical, for example, the coronary arteries, it is possible that permanent, severe damage can occur.

Thus, while conventional stopcocks help to reduce the outflow of blood through angiographic catheters, they are only partially effective. Blood often flows out of a stopcock when opened for a guidewire to pass through it, or when a connection is made to a syringe or an infusion line. Moreover, conventional stopcocks are cumbersome because they often require two hands for operation. One hand holds the stopcock while the other turns the lever. This maneuver may be difficult for a physician to achieve, particularly when, for example, one of the physician's hands is needed to maintain the position of the catheter.

Another problem related to stopcocks arises when a catheter that has been fitted with a stopcock must be attached to a power injector, for example, when a large quantity of x-ray dye is to be injected while obtaining radiographs to visualize the blood vessel. The physician sometimes forgets to turn the stopcock into the opened position, in which case the dye is prevented from entering the catheter. The radiographs are obtained but do not provide the desired diagnostic information because the dye was not injected. The patient is therefore unnecessarily exposed to radiation, and the procedure must be repeated by the physician a second time to obtain the desired diagnostic information.

Apart from conventional stopcocks, other devices are known for reducing blood loss through angiographic catheters. For example, a variety of hemostasis valves are known which may be secured to the proximal end of an angiographic catheter to minimize blood loss around a guidewire that is positioned within a catheter, while permitting fluid to be injected into the catheter with the guidewire in place. One example of such a hemostasis valve is available from Cordis Corporation of Miami, Florida under the product name "Adjustable Hemostasis Valve", Catalog No. 501-622. Such device includes a male luer lock connection fitting for being secured over the proximal end of an angiographic catheter. The device also includes a rotatable barrel which can be manually rotated to adjust the degree of compression exerted upon an annular compression washer. A guidewire may be inserted through the central opening in the rotatable barrel and passed into the proximal end of the catheter. The barrel can then be manually rotated to tighten the compression washer to form a seal about the guidewire. The device also includes a sideport extension with a luer lock fitting to permit flushing and/or infusion of fluids. However, such device must be manipulated by the physician to adjust the degree to which the compression washer seals against the walls of the guidewire. In addition, if the guidewire is to be removed, the device must again be manually rotated, first to release the guidewire, and thereafter to reseal the guidewire passage. As noted above, the physician's hands are often required for other purposes. A similar type of device is also available under the product name "Tuohy-Borst Adapter" from Universal Medical Instrument Corp. of Ballston Spa, New York; the latter device includes a sideport extension through which a high pressure injection of fluid may be made while the guidewire is clamped therein.

Another device which has been introduced to avoid leakage of blood from angiographic catheters is available from Medi-tech, Incorporated of Watertown, Massachusetts under the product name "FloSwitch HP", Catalog No. 44-200. This product resembles the device described within U.S. Patent No. 4,243,034 issued to Brandt, and is further described in Widlus, "Technical Note: Safety of High Pressure Injections Through a Flow Switch Stopcock", CardioVascular and Interventional Radiology, (1988) 11:307-308. The device includes a male luer connector at one end for being secured to the proximal end of the catheter, and includes an opposing end provided with a female luer connector for allowing a syringe to be secured thereto. The device includes a sliding thumb switch which may be opened or closed with or without a guidewire in place. If a guidewire is passed through such device, the slide switch may be advanced toward the closed position until the movable sealing member engages the wall of the guidewire to minimize blood leakage. While representing an improvement over a conventional stopcock or simple hemostasis valve, the "FloSwitch HP" flow control device still requires manual operation by the physician, and still allows a physician to forget that he has left the switch in the closed position when attempting to inject dye into the catheter.

Hemostasis valves using deformable elastomeric seals are widely known for use within catheter introducer sheaths. Such catheter introducer sheaths are available from a variety of manufacturers, including the catheter sheath introducer available from Cordis Corporation of Miami, Florida under the product name "Catheter Sheath Introducer", Catalog No. 501-675U. Patents generally directed to such catheter introducer sheaths include U.S. Patent No. 4,610,665 (Matsumoto et al.); No. 4,626,245 (Weinstein); No. 4,000,739 (Stevens); No. 4,430,081 (Timmermans); and No. 4,673,393 (Suzuki et al.). while such introducer sheaths are effective in preventing blood loss which would otherwise result when a catheter or guidewire is inserted therethrough, such devices do not solve the problem of preventing blood loss from the proximal end of an angiographic catheter of the type used to inject fluids under high pressure within the vascular system.

Finally, a variety of devices are known which include deformable elastomeric seals that are adapted to form a fluid seal but which are deformed upon insertion of the tip of a syringe or other conical member to permit fluid flow therethrough. Examples of such devices are described within U.S. Patent No. 3,837,381 (Arroyo); No. 4,143,853 (Abramson); No. 4,387,879 (Tauschinski); No. 4,765,588 (Atkinson); No. 4,842,591 (Luther); U.S. Patent No. 5,156,596 (Balbierz et al.) and United Kingdom Patent No. 2,067,075 (Krutten et al.). In particular, the patent to Abramson describes a check valve for use with a catheter or needle and including a slit rubber disc supported in a housing having a first male connection having a standard luer-type taper and having a second luer-type female connection. The valve device is described for use in conjunction with a needle having a luer-type female fitting for withdrawing blood samples. Blood samples are withdrawn by inserting into the female connection of the check valve the tip of a syringe having a male connector at its tip to spread apart the slit in the valve. However, no suggestion is made in such disclosure of the use of such a check valve in conjunction with an angiographic catheter, or for passage of a guidewire therethrough.

The above-mentioned patent to Tauschinski discloses a self-sealing connector for coupling a vein catheter to a supply of blood or parenteral solution. The described device includes a slit elastic disc to selectively seal the flow of fluid. In at least one embodiment, an axially slidable member having a central bore is advanced into contact with the slit disc to open the same when the end of a supply hose is inserted into one of the ports of the device. Once again, no suggestion is made that such a device is capable of being used in conjunction with an angiographic catheter, that a guidewire be passed through the elastic disc, or that such a device is adapted for high pressure injections of fluid into an angiographic catheter.

The above-mentioned patent to Atkinson discloses a check valve for coupling a syringe to a fluid supply container for allowing a user to withdraw fluid from the supply container into the syringe. The check valve includes a slit elastomeric diaphragm. Insertion of the distal end of the syringe into the check valve causes such distal end to push through the slit diaphragm.

The above-mentioned patent to Luther describes a one-way valve connector for coupling a syringe tip to a catheter. The one-way valve includes a resilient slit septum that can be deformed by a movable plug. Insertion of the syringe tip causes the plug to move forwardly to deform the septum for allowing liquid to be injected therethrough. Again, no suggestion is made that such a check valve may be used with an angiographic catheter, or that a guidewire be passed through the slit of the resilient septum.

The above-mentioned patent US-A-5 156 596 discloses features a), c) and d) of appended claim 1.

In addition, U.S. Patent No. 3,601,151 to Winnard discloses a one way valve which, in one embodiment, is coupled between a needle inserted into a vein and a syringe used to withdraw blood samples. Such device does not include a slit seal and is not adapted to permit passage of a guidewire therethrough.

Accordingly, it is an object of the present invention to provide a device which can be secured to an angiographic catheter to prevent and/or minimize the loss of blood from the proximal end of such catheter following placement of the catheter within a blood vessel.

Another object of the present invention is to minimize blood loss from an angiographic catheter when inserting a guidewire therein.

Another object of the present invention is to provide such a device which permits a guidewire to be freely passed through an angiographic catheter while providing a seal around the guidewire to prevent any blood from passing between the guidewire and catheter while the guidewire is present.

Another object of the present invention is to prevent or minimize blood loss from an angiographic catheter when connecting or disconnecting a syringe or infusion line thereto.

Still another object of the present invention is to provide such a device which permits a syringe to be coupled to the proximal end of the catheter while effecting a fluid-tight, high-pressure seal therebetween.

Yet another object of the present invention is to provide such a device which does not require any manipulation of levers or switches in order to pass a guidewire into the proximal end of the catheter, and which similarly does not require any manipulation of levers or switches in order to inject or aspirate a fluid therethrough.

A further object of the present invention is to provide such a device which prevents heparinized saline or other anticoagulant fluid from being flushed out of the catheter when a syringe or fluid line is uncoupled from the proximal end of the catheter or when a guidewire is inserted into the catheter.

A still further object of the present invention to provide such a device which is of simple and inexpensive construction.

Another object of the present invention is to provide such a device which automatically opens when a syringe or other medical injection line is attached to it; similarly, when the syringe or injection line is removed therefrom, the device automatically closes without any intervention by the physician.

These and other objects of the invention will become more apparent to those skilled in the art as the description of the invention proceeds.

### Disclosure of Invention

Briefly described, and in accordance with a preferred embodiment thereof, the present invention is a self-sealing valve device for use with angiographic catheters, and including a housing having a first end adapted to form a luer lock connection with the proximal end of the angiographic catheter for forming a fluid tight coupling therewith. The housing includes a second opposing end including a female luer lock connection fitting adapted to receive a complementary male luer lock connection fitting of a syringe, stopcock, or the like, to form a fluid tight connection therewith. The housing includes a central bore extending between the first and second opposing ends along a longitudinal axis of said housing. A deformable elastomeric seal is supported within the housing and extends across the central bore thereof to selectively seal the central bore. The deformable elastomeric seal includes a slit substantially aligned with the longitudinal axis of said housing to permit a guidewire to be passed therethrough while sealingly engaging the guidewire to prevent blood, heparinized saline, or other fluid within the catheter from passing beyond the deformable elastomeric seal.

As described above, the second end of the housing is provided with a female luer lock connection fitting for receiving a mating male luer lock connector. The self-sealing valve device of the present invention further includes a mechanism for deforming the aforementioned elastomeric seal automatically upon coupling of a male luer lock connection fitting to the second end of the housing in order to permit passage of blood or other fluids through the deformable elastomeric seal and through the central bore of the housing following connection of a syringe, stopcock, or the like, to the second end of the housing. In one embodiment of the present invention, this function is achieved by positioning the deformable elastomeric seal within the housing at a distance from the second end of the housing commensurate with the distance by which the conical tip of the syringe or other instrument extends into the second end of the housing for causing the conical tip of the syringe, stopcock, or the like, to directly contact and deform the deformable elastomeric seal in order to permit the passage of fluids therethrough. A guidance member may be provided for use in conjunction with such a device for guiding and supporting a guidewire to be passed through the slit of the deformable elastomeric seal.

In a second embodiment, the mechanism for deforming the elastomeric seal includes a depressor member supported within the housing for movement along the longitudinal axis of the housing between the seal and the second end of the housing. The depressor member has a central bore extending therethrough concentric with the longitudinal axis of the housing for permitting the passage of a guidewire or fluids therethrough. The depressor member has a first end disposed proximate the deformable elastomeric seal for contacting and deforming the seal when urged thereagainst. The depressor member also includes a second opposing end disposed proximate the second end of the housing and having an inwardly tapering conical surface for being abutted by the conical tip of a syringe, stopcock, or the like, connected to the second end of the housing. The insertion of the conical tip of the syringe, stopcock, or the like causes the depressor member to advance toward the deformable elastomeric seal to break the seal to permit fluids to pass therethrough. Preferably, the central bore of the housing includes a reduced-diameter portion proximate to the deformable elastomeric seal and disposed between said deformable elastomeric seal and the first end of the housing. The reduced-diameter portion provides a shoulder against which the deformable elastomeric seal rests when deformed, the shoulder thereby preventing excessive deformation of the deformable elastomeric seal.

Another aspect of the present invention relates to the incorporation of such a self-sealing valve device within the proximal end of an angiographic catheter as a unitary device ready for use.

Yet another aspect of the present invention is a method of performing an angiographic procedure using an angiographic catheter wherein the distal end of an angiographic catheter is placed within a blood vessel of a body and a deformable elastomeric seal having a slit formed therein is provided substantially adjacent the proximal end of the catheter to selectively seal the proximal end of the catheter against the loss of blood or other fluids therefrom. A fluid-tight coupling is then formed between the tip of a syringe and the proximal end of the catheter, while simultaneously deforming the deformable elastomeric seal to permit fluid to be passed therethrough or blood to be aspirated therethrough. Fluid is injected under pressure from the syringe through the deformable elastomeric seal and into the catheter for introducing the fluid under pressure into the body. The syringe is uncoupled from the proximal end of the catheter, while permitting the deformable elastomeric seal to return to its original position sealing the proximal end of the catheter. The step of providing the deformable elastomeric seal is preferably achieved by providing a housing having first and second opposing ends and including a central bore extending therebetween, supporting the deformable elastomeric seal within the housing and extending across the central bore, and forming a luer lock connection between a first end of the housing and the proximal end of the catheter. The step of forming a fluid-tight coupling between the tip of a syringe and the proximal end of the catheter is preferably achieved by forming a luer lock connection between the tip of the syringe and the second end of the housing.

The method of the present invention may also include the further step of inserting a guidewire through both the proximal end of the catheter and the slit of the deformable elastomeric seal, and passing the guide wire into the body through the catheter in order to position the guidewire within a blood vessel of the body.

### Brief Description of the Drawings

Fig. 1 is a perspective view of a self-sealing valve device for use with an angiographic catheter in accordance with the present invention, and further partially illustrates the proximal end of an angiographic catheter as well as the distal tip of a syringe.
Fig. 2 is a sectional view of the components shown in Fig. 1 viewed through the plane designated by arrows 2-2 as shown in Fig.1.
Fig. 3 is a sectional view like that of Fig. 2 but directed to an alternate embodiment of the present invention, and including a guidance member for guiding a guidewire for passage through a deformable seal of the valve device.
Fig. 4 is a sectional view of the self-sealing valve device shown in Fig. 3 wherein the distal tip of a syringe engages and deforms the elastomeric seal of the valve device.
Figs. 5A-5G illustrate steps of a method for performing an angiographic procedure.
Fig. 5A illustrates a guidewire inserted within a blood vessel of a body.
Fig. 5B illustrates an angiographic catheter equipped with a self-sealing valve device being guided over the guidewire into the blood vessel.
Fig. 5C illustrates the guidewire being removed from the angiographic catheter.
Fig. 5D illustrates a syringe being coupled by a luer lock connection fitting with the self-sealing valve device for injecting fluid into the catheter.
Fig. 5E illustrates the syringe being detached from the valve device.
Fig. 5F illustrates the guidewire being passed into the valve device and through the catheter into the blood vessel.
Fig. 5G illustrates the angiographic catheter being removed from the guidewire.

### Best Mode for Carrying Out the Invention

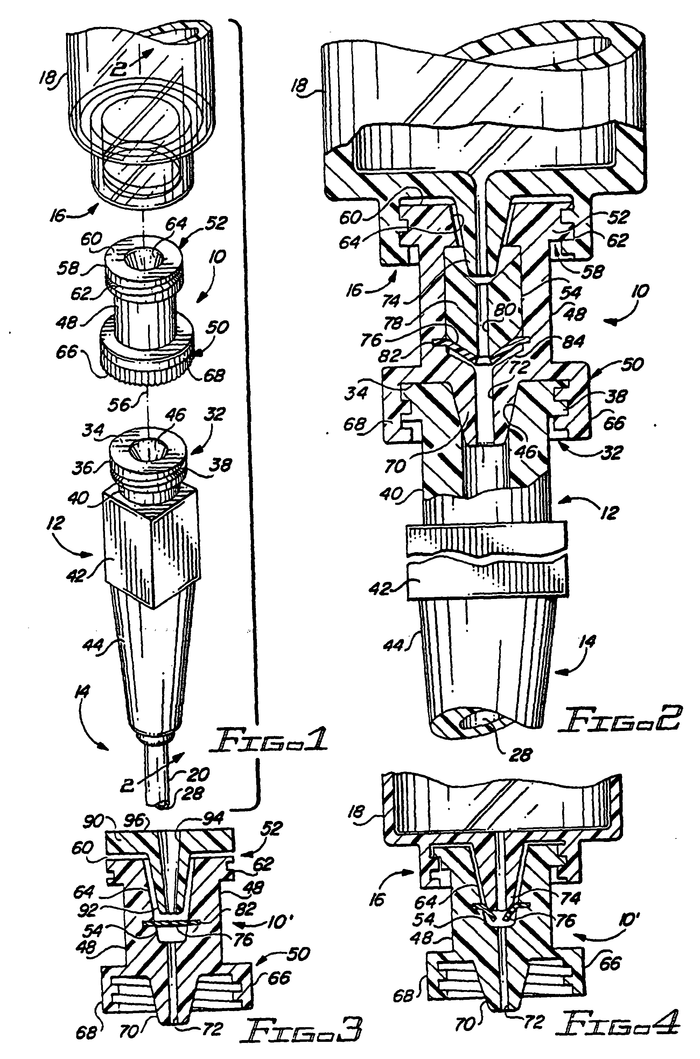

Within Fig. 1, a self-sealing valve device, constructed in accordance with one embodiment of the present invention, is designated generally by reference numeral 10. Also shown in Fig. 1 is the proximal end 12 of an angiographic catheter 14, as well as the distal tip 16 of a syringe 18.

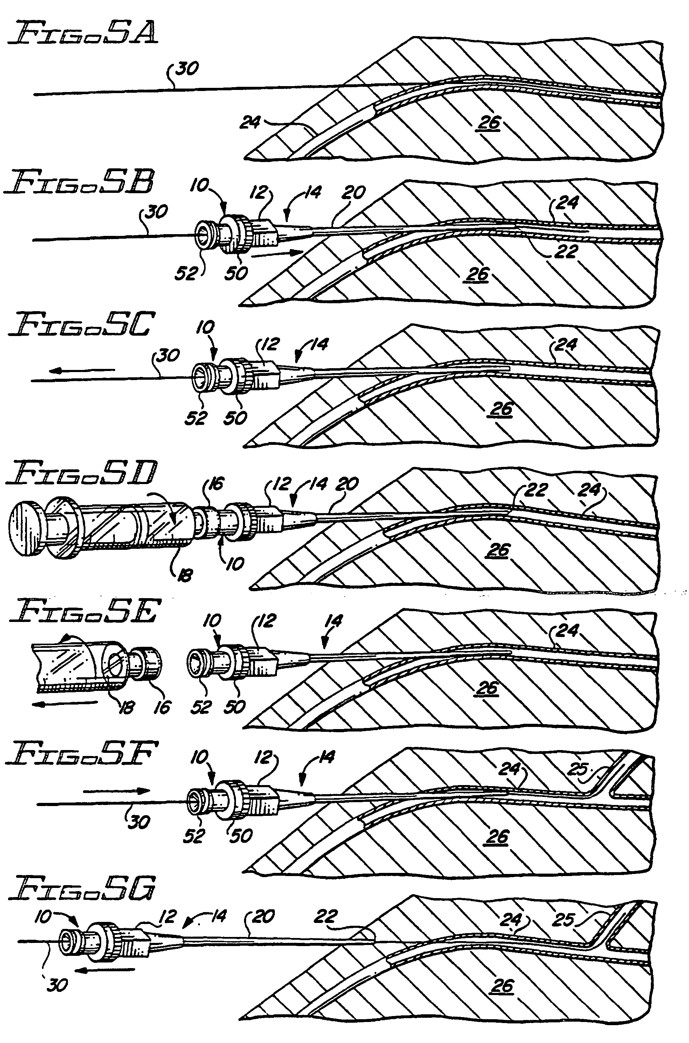

Before further describing self-sealing valve device 10, it will be helpful to first describe the structure of angiographic catheter 14. Catheter 14 includes a central, flexible shaft 20 which extends between proximal end 12 and an opposing distal end; this opposing distal end is visible within, for example, Fig. 5D and is designated therein by reference numeral 22. As indicated within Fig. 5D, distal end 22 of angiographic catheter 14 is adapted to be inserted into a blood vessel 24 of a patient's body 26. As indicated in Figs. 1 and 2, a lumen 28 extends continuously through catheter 14 from proximal end 12 to distal end 22. Lumen 28 allows fluids to be passed through catheter 14, and also is adapted to receive a guidewire 30, as shown in Fig. 5B, to properly position catheter 14 within body 26.

Returning to Figs. 1 and 2, proximal end 12 of angiographic catheter 14 includes a female luer lock connection fitting 32 which terminates in an annular sealing surface 34. Extending below sealing surface 34 is a cylindrical surface 36 upon which a single raised thread 38 is formed; raised thread 38 gradually spirals upwardly toward annular sealing surface 34. A reduced diameter cylindrical portion 40 couples female luer lock connection fitting 32 to squared portion 42, adapted to be grasped by a physician to steady catheter 14 when making or removing connections to luer lock connector 32. Conically tapered portion 44 of proximal end 12 joins with the upper end of shaft 20 of catheter 14.

As shown in Figs. 1 and 2, female luer lock connector 32 of angiographic catheter 14 includes an inwardly converging conical bore 46 which extends through the center of annular sealing surface 34 and which communicates with lumen 28. Female luer lock connector 32 is adapted to receive a complimentary male luer lock connection fitting to form a fluid tight connection therewith; such a complimentary male luer lock connection fitting may be provided by, for example, distal end 16 of syringe 18, or by a port of a conventional stopcock, an infusion line, or the like. The features of angiographic catheter 14, including the structure of female luer lock connector 32 are well known in the art and do not form part of the present invention. However, an understanding of such features is helpful to an understanding the features of the present invention, which will now be described in greater detail.

Referring to Figs. 1 and 2, self-sealing valve device 10 includes a housing 48, which is preferably molded from a medical-grade plastic similar to materials commonly used, for example, to mold proximal end 12 of catheter 14. Housing 48 includes a first end 50 and an opposing second end 52. A central bore 54 (see Fig. 2) extends continuously between first end 50 and second end 52 of housing 48 along longitudinal axis 56 of housing 48.

Second end 52 of housing 48 includes a female luer lock connection fitting 58 substantially identical in structure to female luer lock connector 32 described above in conjunction with the proximal end 12 of catheter 14. Female luer lock connector 58 includes an annular sealing surface 60, a raised spiral thread 62, and an inwardly converging conical bore 64 in fluid communication with central bore 54 of housing 48. Like female luer connector 32, female luer connector 58 is adapted to receive a complimentary male luer lock connection fitting of a syringe, stopcock, infusion line or the like, to form a fluid-tight connection therewith.

As shown in Figs. 1 and 2, first end 50 of housing 48 includes a male luer lock connection fitting 66 adapted to be releasably secured with female luer lock connector 32 of catheter 14 in order to form a fluid-tight coupling therebetween. Male luer lock connector 66 includes a cylindrical extension 68 which preferably has a knarled outer surface, as shown in Fig. 1, for allowing a physician to more easily grip and rotate valve device 10 when securing the same to the proximal end 12 of catheter 14. As shown best in Fig. 2, cylindrical extension 68 is internally threaded for releasably receiving raised thread 38 of female luer lock connector 32. Male luer lock connector 50 further includes a conically-tapered tip portion 70 adapted to extend within conical bore 46 of female luer lock connector 32. The degree of taper of conical portion 70 is identical to that of conical bore 46 in order to effect a fluid-tight seal between male luer lock connector 50 and female luer lock connector 32 when such components are threaded together. Conical tip portion 70 includes a central bore 72 extending concentric with bore 54 for permitting fluids and/or a guidewire to be passed therethrough.

The male lock luer connector fitting 16 of syringe 18 essentially has the same features as those described immediately above for male lock luer connector 50. In particular, male lock luer connector 16 of syringe 18 includes a conically tapered tip portion 74 adapted to extend through, and sealingly engage, conical bore 64 of female luer lock connector 58.

As shown in Fig. 2, valve device 10 further includes a deformable elastomeric seal 76 supported within housing 48 and extending across central bore 54 thereof. Deformable elastomeric seal 76 includes a central slit formed therein substantially aligned with longitudinal axis 56 of housing 48. Deformable elastomeric seal 76 ordinarily extends continuously across the central bore within valve device 10 to selectively seal such central bore (see Fig. 3). The central slit formed within deformable elastomeric seal 76 permits a guidewire to be passed therethrough while sealingly engaging the walls of the guidewire to prevent blood or other fluids from passing around the guidewire through central bore 54. When the guidewire is removed, deformable elastomeric seal 76 returns to the closed position (see Fig. 3) for sealing the central bore of valve device 10. Those skilled in the art will appreciate that the slit formed within deformable elastomeric seal 76 can be a single linen slit or two or more slits which intersect one another proximate the center of elastomeric seal 76. Likewise, those skilled in the art will appreciate that deformable elastomeric seal 76 can comprise a single elastomeric disc or two or more slit elastomeric disks disposed one next to the other. In this regard, the reader is directed to the elastomeric seal structures shown in the aforementioned U.S. Patent Nos. 4,387,879 (Tauschinski); 4,610,665 (Matsumoto et al.); 4,626,245 (Weinstein); and 4,673,393 (Suzuki et al.), the specifications and drawings of which patents are herein incorporated by reference. Those skilled in the art will appreciate that the term "deformable elastomeric seal", as used herein, is intend to include all such slit seal structures.

When syringe 18 is to be coupled to catheter 14 in order to inject or aspirate fluid, it is desirable that deformable elastomeric seal 76 be opened to facilitate the flow of fluid through valve device 10. In a first embodiment shown in Fig. 2, valve device 10 includes a depressor member 78 of generally cylindrical shape supported within central bore 54 of housing 48 for limited movement therein along longitudinal axis 56. As shown in Fig. 2, depressor member 78 is positioned between deformable elastomeric seal 76 and second end 52 of housing 48. Depressor member 78 has a central bore 80 formed therein of sufficient diameter to permit the passage of a guidewire, or the passage of fluid, therethrough. Central bore 80 of depressor member 78 is concentric with longitudinal axis 56 of housing 48. Depressor member 78 includes a generally bullet-shaped lower tip at a first end thereof, disposed proximate deformable elastomeric seal 76, for selectively contacting and deforming deformable elastomeric seal 76 when urged thereagainst, as shown in Fig. 2. Depressor member 78 also includes a second opposing end disposed proximate second end 52 of housing 48 and having an inwardly tapering conical surface for being abutted by conical tip 74 of syringe 18. As shown in Fig. 2, coupling of male luer lock connector 16 of syringe 18 to female luer connector 58 of housing 48 causes conical tip 74 of syringe 18 to push against depressor member 78, and thereby automatically cause deformable elastomeric seal 76 to open. When syringe 18 is removed from valve device 10, the natural resiliency of deformable elastomeric seal 76 urges depressor member 78 upwardly toward second end 52 of housing 48, thereby allowing elastomeric seal 76 to return its closed position (see Fig. 3). Thus, depressor member 78 serves as a means for deforming deformable elastomeric seal 76 upon coupling of a male luer lock connector to second end 52 of housing 48 in order to permit the passage of blood or other fluids through deformable elastomeric seal 76.

A second embodiment of the self-sealing valve device of the present invention is shown in Figs. 3 and 4, and is designated generally by reference numeral 10'. Those features and components of valve device 10' which correspond with features described above in conjunction with valve device 10 are designated by like reference numerals. As shown in Fig. 3, no depressor member is required. Housing 48 of valve device 10' includes an inwardly tapering conical bore 64 that is continuous with central bore 54 of housing 48. In addition, deformable elastomeric seal 76 is supported much more closely to second end 52 of housing 48 as compared with valve device 10 shown in Fig. 2. As shown in Fig. 4, coupling of syringe 18 to second end 52 of valve device 10' causes conical tip 74 of syringe 18 to extend sufficiently within conical bore 64 to directly contact and deform deformable elastomeric seal 76. Deformable elastomeric seal 76 is supported within conical bore 54/64 at a distance from second end 52 corresponding to the distance by which conical tip 74 extends within second end 52. Accordingly, in this embodiment, no depressor member is required because the conical tip of the syringe, stopcock or other male luer lock connector device coupled to second end 52 directly contacts deformable elastomeric seal 76. Once syringe 18 is removed from valve device 10'; deformable elastomeric seal 76 returns to its closed position shown in Fig. 3.

Within valve device 10', deformable elastomeric seal 76 is supported within a circular recess 82 formed within conical bore 54/64. Thus, within the embodiment of the present invention shown in Figs. 3 and 4, the circular recess 82 which supports deformable elastomeric seal 76 at a predetermined distance from second end 52 of housing 48 serves as a means for causing deformable elastomeric seal 76 to be deformed upon coupling of a male luer lock connector to second end 52 of housing 48.

Within the embodiment of the invention shown in Fig. 2, the inwardly tapering conical surface at the upper end of depressor member 78 aids in properly centering a guidewire being inserted through second end 52 of valve device 10. Depressor member 78 also helps to maintain the rigidity of the guidewire as it passes through and breaks the seal of deformable elastomeric seal 76. In contrast, valve device 10' shown in Figs. 3 and 4 does not include a means for stiffening or centering a guidewire to facilitate passage of the guidewire through deformable elastomeric seal 76. Accordingly, as shown in Fig. 3, a guidance member 90 may be optionally used for such purpose. Guidance member 90 includes a conical tip 92 somewhat resembling the conical tip 74 of syringe 18. Guidance member 90 further includes a central bore 94, the diameter of which is somewhat greater adjacent upper end 96 then at the lower end of conical tip 92. The larger diameter opening adjacent upper end 96 facilitates the threading of the guidewire therethrough, while the reduced diameter portion extending through conical tip 92 helps to rigidify and center the distal tip of the guidewire directly over the slit portion of the deformable elastomeric seal 76. Conical tip 92 has a taper closely resembling the taper of conical bore 64 for causing central bore 94 to be substantially aligned with the longitudinal axis of housing 48 to guide the distal tip of the guidewire toward and through the slit formed within deformable elastomeric seal 76. After the distal tip of the guidewire is passed through deformable elastomeric seal 76, guidance member 90 may be removed by pulling guidance member 90 over the external (proximal) end of the guidewire.

The embodiments of the present invention described above with reference to Figs. 1-4 contemplate self-sealing valve device 10 and 10' as a components separate and apart from angiographic catheter 14. However, in a related aspect of the present invention, the self-sealing valve device may be integrally incorporated within the proximal end of an angiographic catheter, if desired. Essentially, this modification involves merging second end 50 of valve device 10 with proximal end 12 of angiographic catheter 14 to form an integral unified structure. Within such modified catheter structure, lumen 28 may essentially be continuous with central bore 72 of valve device 10. Such a modified form of angiographic catheter could be provided either using a depressor member 78, as shown in Fig. 2, or omitting such depressor member, as shown in Figs. 3 and 4.

As indicated in Fig. 2, deformable elastomeric seal 76 may be supported within a circular recess 82 formed within the internal wall of central bore 54. Preferably, central bore 54 includes a reduced diameter portion 84 immediately below deformable elastomeric seal 76; reduced diameter portion 84 provides a shoulder against which deformable elastomeric seal 76 rests when deformed, thereby preventing excessive deformation of deformable elastomeric seal 76.

Another aspect of the present invention relates to the method of performing an angiographic procedure using an angiographic catheter in the manner described above. Such method is best described with reference to Figs. 5A through 5G. In Fig. 5A, a guidewire 30 is shown after the distal portion thereof has been inserted percutaneously into a blood vessel 24 of a patient's body 26. Within Fig. 5B, the distal portion of catheter 14 is advanced over guidewire 30 into blood vessel 24, the distal end of catheter 14 being designated therein by reference numeral 22. As shown in Fig. 5B, valve device 10 has already been secured to the proximal end 12 of catheter 14, and guidewire 30 extends through the slit of the deformable elastomeric seal therein. Accordingly, no blood escapes from second end 52 of valve device 10 as catheter 14 is being placed within blood vessel 24.

Having advanced catheter 14 to the desired point within blood vessel 24, guidewire 30 may then be removed, as indicated in Fig. 5C. As soon as guidewire 30 exits the deformable elastomeric seal within valve device 10, the seal returns to its closed position, preventing any loss of blood from second end 52 of valve device 10. Turning to Fig. 5D, the male luer lock connector 16 of syringe 18 is secured over the female luer lock connector at the second end 52 of valve device 10 to form a fluid-tight coupling between the tip of the syringe and proximal end 12 of catheter 14. As explained above, this operation causes the deformable elastomeric seal to be deformed to permit fluid within syringe 18 to be injected into catheter 14. After injecting such fluid, for example, an angiographic dye used to perform diagnostic testing, syringe 18 is unthreaded from second end 52 of valve device 10. During this procedure the deformable elastomeric seal within valve device 10 returns to its closed position to prevent the escape of blood or other fluids within catheter 14.

Such diagnostic testing may reveal that catheter 14 must be moved to a different portion of the vascular system, or perhaps, replaced with a different catheter. In this instance, guidewire 30 may be reinserted into valve device 10 and through catheter 14 in order to place the distal tip of guidewire 30 within a branch blood vessel 25, as shown in Fig. 5F. Again, the deformable elastomeric seal within valve device 10 enters into sealing engagement with the walls of guidewire 30 to prevent the passage of blood or other fluid out of second end 52 of valve device 10. If appropriate, catheter 14 may either be advanced over guidewire 30 to the branch vessel 25, or catheter 14 may be removed over guidewire 30, as shown in Fig. 5G, and be replaced with another catheter that is then passed over guidewire 30 into branch vessel 25.

Those skilled in the art will now appreciate that an improved self-sealing valve device has been described for use with angiographic catheters. The described valve device prevents or minimizes the loss of blood from the catheter when the catheter is inserted within the vascular system while permitting a guidewire to be freely passed into the catheter. The valve device does not require any levers, switches, or other manually operated components to open and close the seal therein, and automatically opens upon coupling of a syringe, infusion line, stopcock, or other medical instrument including a male luer lock connector.

While the invention has been described with reference to preferred embodiments thereof, the description is for illustrative purposes only and is not to be construed as limiting the scope of the invention. Various modifications and changes may be made by those skilled in the art without departing from the scope of the invention as defined by the appended claims.

## Claims

1. A self-sealing valve device (10) for an angiographic catheter (14), the catheter including a distal end (22) for insertion into a blood or lymph-vessel of the body and an opposing proximal end (12), the catheter including a lumen (28) extending between the distal and proximal ends for passing fluids therethrough and for receiving a flexible guide wire (30) used to position the catheter in the body, the proximal end of the catheter including a female luer lock connection fitting (32) adapted to receive a complementary male luer lock connection fitting (16) of a syringe (18), stopcock, or the like, to form a fluid tight connection therewith, said self-sealing valve device including:
a) a housing (48) having a central bore (54) extending between first (50) and second (52) opposing ends of said housing along a longitudinal axis of said housing;
b) said first end (50) of said housing (48) including a male luer lock connection fitting (66) for forming a fluid tight coupling with the female luer lock connection fitting (32) at the proximal end (12) of the catheter (14);
c) said second end of said housing including a female luer lock connection fitting (58) adapted to receive a complementary male luer lock connection fitting (16) of a syringe (18), stopcock, or the like, to form a fluid tight connection therewith, said female luer lock connection fitting including an inwardly converging conical bore (64) for receiving a conically-shaped tip (74) of a syringe, stopcock, or the like;
d) a deformable elastomeric seal (76) supported within said housing and extending across the central bore thereof to selectively seal the central bore of said housing, said deformable elastomeric seal including at least one slit substantially aligned with the longitudinal axis of said housing to permit the flexible guidewire to be passed therethrough while sealingly engaging the flexible guidewire to prevent blood inside the catheter from passing beyond said deformable elastomeric seal, the deformable elastomeric seal being opened upon coupling of a male luer lock connection fitting (16) to said second end of said housing to permit passage of blood or other fluids through the deformable elastomeric seal and through the central bore of said housing following connection of a syringe, stopcock, or the like, to said second end of said housing; and
e) guide means disposed generally proximate the deformable elastomeric seal and having a central bore (80/94) formed therein, said central bore being substantially concentric with the longitudinal axis of said housing, said central bore having a diameter which allows the flexible guidewire (30) to be passed through the catheter for centering the flexible guidewire and for stiffening the flexible guidewire as it passes through said deformable elastomeric seal.

2. The self-sealing valve device for an angiographic catheter as recited by claim 1, wherein said deformable elastomeric seal (76) is positioned within said housing at a distance from said second end (52) of said housing, whereby said distance corresponds to a predetermined length by which a conical tip (16) of a syringe (18), stopcock, or the like, extends into said female luer lock connection fitting (58) of said second end (52) of said housing (48), for causing the conical tip of the syringe, stopcock, or the like, to directly contact and deform said deformable elastomeric seal in order to permit the passage of fluids therethrough.

3. The self-sealing valve device for an angiographic catheter as recited by Claim 2 wherein said guide means is a guidance member (90) for facilitating passage of the distal tip of the guidewire through said at least one slit of said deformable elastomeric seal, said guidance member having a conical tip (92) received by said second end of said housing, said conical tip of said guidance member including a bore (94) extending therethrough commensurate in diameter with the diameter of the guidewire, the bore of said guidance member being substantially aligned with the longitudinal axis of said housing, and the conical tip of said guidance member extending proximate said deformable elastomeric seal for guiding the distal tip of a guidewire toward and through said at least one slit of said deformable elastomeric seal and into the catheter.

4. The self-sealing valve device for an angiographic catheter as recited by Claim 1 wherein said guide means includes a depressor member (78) movably supported within said housing between said deformable elastomeric seal and second end of said housing, said depressor member having a central bore (80) extending therethrough for permitting the passage of a guidewire or fluids therethrough, the central bore of said depressor member being concentric with the longitudinal axis of said housing, said depressor member being supported for movement along the longitudinal axis of said housing, said depressor member having a first end disposed proximate said deformable elastomeric seal for contacting and deforming said deformable elastomeric seal when urged thereagainst, said depressor member further including a second opposing end disposed proximate the second end of said housing and having an inwardly tapering conical surface for being abutted by the conical tip of a syringe, stopcock, or the like, connected to said second end of said housing, insertion of the conical tip of the syringe, stopcock, or the like causing said depressor member to advance toward said deformable elastomeric seal to break the seal to permit fluids to pass therethrough.

5. The self-sealing valve device for an angiographic catheter as recited by Claim 4 wherein the central bore (54) of said housing (48) includes a reduced diameter portion (84) proximate to said deformable elastomeric seal disposed between said deformable elastomeric seal and the first end of said housing, said reduced-diameter portion providing a shoulder against which said deformable elastomeric seal rests when deformed, said shoulder preventing excessive information of said deformable elastomeric seal.

6. The self-sealing valve device for an angiographic catheter as recited by claim 1 further including:
(a) an angiographic catheter (14) including a distal end (22) for insertion into a blood or lymph vessel of the body and an opposing proximal end (12), said angiographic catheter including a lumen (28) extending between the distal and proximal ends for passing fluids therethrough and for receiving a guidewire (30) used to position said catheter in the body, the proximal end of said catheter including a female luer lock connection (32) secured to said male luer lock connection fitting (66) provided at said first end (50) of said housing (48) to form a fluid tight coupling therewith.

7. The self-sealing valve device for an angiographic catheter as recited by claim 6 wherein the conical tip (74) of a syringe (18), stopcock, or the like, extends for a predetermined length into said female luer lock connection fitting (58) of said second end (52) of said housing (48), and wherein said deformable elastomeric seal (76) is positioned within said housing at a distance from said second end of said housing of approximately said predetermined length for causing the conical tip of the syringe, stopcock, or the like, to directly contact and deform said deformable elastomeric seal in order to permit the passage of fluids therethrough.

8. The self-sealing valve device recited by Claim 7 wherein said guide means is a guidance member (90) for facilitating passage of the distal tip of the guidewire (30) through said at least one slit of said deformable elastomeric seal, said guidance member having a conical tip (92) received by said second end (52) of said housing, said conical tip of said guidance member including a bore (94) extending therethrough commensurate in diameter with the diameter of the guidewire, the bore of said guidance member being substantially aligned with the longitudinal axis of said housing, and the conical tip of said guidance member extending proximate said deformable elastomeric seal for guiding the distal tip of a guidewire toward and through said at least one slit of said deformable elastomeric seal and into said catheter.

9. The self-sealing valve device as recited by Claim 6 wherein said guide means includes a depressor member (78) movably supported within said housing between said deformable elastomeric seal and second end of said housing, said depressor member having a central bore (80) extending therethrough for permitting the passage of a guidewire (30) or fluids therethrough, the central bore of said depressor member being concentric with the longitudinal axis of said housing, said depressor member being supported for movement along the longitudinal axis of said housing, said depressor member having a first end disposed proximate said deformable elastomeric seal for contacting and deforming said deformable elastomeric seal when urged thereagainst, said depressor member further including a second opposing end disposed proximate the second end of said housing and having an inwardly tapering conical surface for being abutted by the conical tip of a syringe, stopcock, or the like, connected to said second end of said housing, insertion of the conical tip of the syringe, stopcock, or the like causing said depressor member to advance toward said deformable elastomeric seal to break the seal to permit fluids to pass therethrough.

10. The self-sealing valve device as recited by Claim 9 wherein the central bore (54) of said housing (48) includes a reduced-diameter portion (84) proximate to said deformable elastomeric seal (76) disposed between said deformable elastomeric seal and the first end of said housing, said reduced-diameter portion providing a shoulder against which said deformable elastomeric seal rests when deformed, said shoulder preventing excessive deformation of said deformable elastomeric seal.

## Patentansprüche

1. Selbstschließende Ventilvorrichtung (10) für einen angiographischen Katheter (14), wobei der Katheter ein distales Ende (22) zur Einführung in ein Blut- oder Lymphgefäß des Körpers und ein gegenüberliegendes proximales Ende (12) aufweist, der Katheter ein Lumen (28) aufweist, das zwischen dem distalen und proximalen Ende verläuft, um Fluide durchfließen zu lassen, und um einen flexiblen Führungsdraht (30) aufzunehmen, der verwendet wird, um den Katheter im Körper zu positionieren, wobei das proximale Ende des Katheters ein negatives Luer-Anschlussstück (32) aufweist, um ein komplementäres positives Luer-Anschlussstück (16) einer Spritze (18), eines Absperrhahns oder dergleichen aufzunehmen, um eine fluiddichte Verbindung zwischen ihnen auszubilden, und die selbstschließende Ventilvorrichtung aufweist:
(a) ein Gehäuse (48) mit einer zentralen Bohrung (54), die zwischen ersten (50) und zweiten (52) gegenüberliegenden Enden des Gehäuses entlang einer Längsachse des Gehäuses verläuft;
(b) das erste Ende (50) des Gehäuses (48), ein positives Luer-Anschlussstück (66) aufweist, um eine fluiddichte Verbindung mit dem negativen Luer-Anschlussstück (32) am proximalen Ende (12) des Katheters (14) auszubilden;
(c) das zweite Ende des Gehäuses ein negatives Luer-Anschlussstück (58) aufweist, das so ausgestaltet ist, um ein komplementäres positives Luer-Anschlussstück (16) einer Spritze (18), eines Absperrhahns oder dergleichen aufzunehmen, um zwischen ihnen eine fluiddichte Verbindung auszubilden, und das negative Luer-Anschlussstück eine nach innen konvergierende konische Bohrung (64) zur Aufnahme eines konischen Endes (74) einer Spritze, eines Absperrhahns oder dergleichen aufweist;
(d) eine deformierbare elastomere Abdichtung (76), die innerhalb des Gehäuses getragen wird, und sich über die zentrale Bohrung davon erstreckt, um die zentrale Bohrung des Gehäuses selektiv abzudichten, wobei die deformierbare elastomere Abdichtung mindestens einen Öffnungsspalt aufweist, die im wesentlichen mit der Längsachse des Gehäuses ausgerichtet ist, um es zu ermöglichen, den flexiblen Führungsdraht hindurchzuführen, wobei der flexible Führungsdraht abgedichtet wird, um zu verhindern, das Blut innerhalb des Katheters über die deformierbare elastomere Abdichtung hinausfließt, wobei die deformierbare elastomere Abdichtung beim Anschließen eines positiven Luer-Anschlussstücks (16) an das zweite Ende des Gehäuses geöffnet wird, um einen Durchtritt von Blut oder anderen Fluiden durch die deformierbare elastomere Abdichtung und durch die zentrale Bohrung des Gehäuses nach Verbindung mit einer Spritze, einem Absperrhahn oder dergleichen zum zweiten Ende des Gehäuses zu ermöglichen; und
(e) ein Führungsteil, das der deformierbaren elastomeren Abdichtung im wesentlichen unmittelbar benachbart ist, und das eine darin ausgebildete zentrale Bohrung (80, 94) aufweist, wobei die zentrale Bohrung mit der Längsachse des Gehäuses im wesentlichen konzentrisch verläuft, und die zentrale Bohrung einen Durchmesser aufweist, der es ermöglicht, den flexiblen Führungsdraht (30) durch den Katheter hindurchzuführen, um den flexiblen Führungsdraht zu zentrieren, und um den flexiblen Führungsdraht zu versteifen, wenn er durch die deformierbare elastomere Abdichtung hindurchläuft.

2. Selbstschließende Ventilvorrichtung für einen angiographischen Katheter nach Anspruch 1, worin die deformierbare elastomere Abdichtung (76) innerhalb des Gehäuses in einer Entfernung vom zweiten Ende (52) des Gehäuses positioniert ist, wobei die Entfernung einer bestimmten Länge entspricht, in der sich ein konisches Ende (16) einer Spritze (18), eines Absperrhahns oder dergleichen in das negative Luer-Anschlussstück (58) des zweiten Endes (52) des Gehäuses (48) erstreckt, damit das konische Ende der Spritze, des Absperrhahns oder dergleichen die deformierbare elastomere Abdichtung direkt kontaktiert und deformiert, um einen Durchfluss des Fluids durch sie zu ermöglichen.

3. Selbstschließende Ventilvorrichtung für einen angiographischen Katheter nach Anspruch 2, worin die Führung ein Führungsteil (90) ist, um einen Durchgang des distalen Endes des Führungsdrahts durch mindestens einen Spalt der deformierbaren elastomeren Abdichtung zu erleichtern, und das Führungsteil ein konisches Ende (92) aufweist, das vom zweiten Ende des Gehäuses aufgenommen wird, das konische Ende des Führungsteils eine hindurchlaufende Bohrung (94) aufweist, die im Durchmesser dem Durchmesser des Führungsdrahts entspricht, wobei die Bohrung des Führungsteils im wesentlichen zur Längsachse des Gehäuses ausgerichtet ist, und das konische Ende des Führungsteils sich zur unmittelbaren Nachbarschaft der deformierbaren elastomeren Abdichtung erstreckt, um das distale Ende des Führungsdrahts gegen und durch den mindestens einen Spalt der deformierbaren elastomeren Abdichtung und in den Katheter zu führen.

4. Selbstschließende Ventilvorrichtung für einen angiographischen Katheter nach Anspruch 1, worin das Führungsteil ein Niederdrückteil (78) aufweist, das innerhalb des Gehäuses zwischen der deformierbaren elastomeren Abdichtung und dem zweiten Ende des Gehäuses beweglich gehalten wird, und das Niederdrückteil eine dadurch verlaufende zentrale Bohrung (80) aufweist, um den Durchgang eines Führungsdrahts oder Fluids zu ermöglichen, die zentrale Bohrung des Niederdrückteils mit der Längsachse des Gehäuses konzentrisch verläuft, das Niederdrückteil zur Bewegung längs der Längsachse des Gehäuses gehalten wird, das Niederdrückteil ein zur deformierbaren elastomeren Abdichtung unmittelbar benachbartes erstes Ende aufweist, um die deformierbare elastomere Abdichtung zu deformieren, wenn es dagegen gedrückt wird, das Niederdrückteil außerdem ein zweites gegenüberliegendes Ende aufweist, das zum zweiten Ende des Gehäuses unmittelbar benachbart ist, und eine sich nach innen verjüngende konische Oberfläche aufweist, an die das mit dem zweiten Ende des Gehäuses verbundene konische Ende einer Spritze, eines Absperrhahns oder dergleichen anstößt, und die Einführung des konischen Endes der Spritze, des Absperrhahns oder dergleichen verursacht, das das Niederdrückteil gegen die deformierbare elastomere Abdichtung vorrückt, um die Abdichtung aufzubrechen, damit Fluide hindurchtreten können.

5. Selbstschließende Ventilvorrichtung für einen angiographischen Katheter nach Anspruch 4, worin die zentrale Bohrung (54) des Gehäuses (48) in unmittelbarer Nachbarschaft zur elastomeren Abdichtung einen Teil (84) mit verringertem Durchmesser aufweist, der zwischen der deformierbaren elastomeren Abdichtung und dem ersten Ende des Gehäuses angeordnet ist, wobei der Teil mit verringertem Durchmesser eine Schulter ergibt, auf der die deformierbare elastomere Abdichtung bei der Deformation aufliegt, wobei die Schulter eine übermäßige Deformation der deformierbaren elastomeren Abdichtung verhindert.

6. Selbstschließende Ventilvorrichtung für einen angiographischen Katheter nach Anspruch 1, die außerdem aufweist:
(a) einen angiographischen Katheter (14) mit einem distalen Ende (22) zur Einführung in ein Blut- oder Lymphgefäß des Körpers und einem gegenüberliegenden proximalen Ende (12), wobei der angiographische Katheter ein Lumen (28) aufweist, das zwischen dem distalen und proximalen Ende verläuft, um Fluide hindurchlaufen zu lassen, und um einen Führungsdraht (30) aufzunehmen, der verwendet wird, um den Katheter im Körper zu positionieren, das proximale Endes des Katheters ein negatives Luer-Anschlussstück (32) aufweist, das an dem ersten Ende (50) des Gehäuses (48) vorgesehenen positiven Luer-Anschlussstück (66) befestigt ist, um damit eine fluiddichte Verbindung auszubilden.

7. Selbstschließende Ventilvorrichtung für einen angiographischen Katheter nach Anspruch 6, worin das konische Ende (74) einer Spritze (18), eines Absperrhahns oder dergleichen mit einer bestimmten Länge in das negative Luer-Anschlussstück (58) des zweiten Endes (52) des Gehäuses (48) verläuft, und worin die deformierbare elastomere Abdichtung (76) innerhalb des Gehäuses in einem Abstand vom zweiten Ende des Gehäuses positioniert ist, der ca. der bestimmten Länge entspricht, wodurch verursacht wird, dass das konische Ende der Spritze, des Absperrhahns oder dergleichen die deformierbare elastomere Abdichtung direkt kontaktiert und deformiert, um dadurch einen Durchgang von Fluiden zu ermöglichen.

8. Selbstschließende Ventilvorrichtung nach Anspruch 7, worin die Führung ein Führungsteil (90) ist, um einen Durchtritt des distalen Endes des Führungsdrahts (30) durch den mindestens einen Öffnungsspalt der deformierbaren elastomeren Abdichtung zu erleichtem, das Führungsteil ein konisches Ende (92) aufweist, das von dem zweiten Ende (52) des Gehäuses aufgenommen wird, das konische Ende des Führungsteils eine hindurchlaufende Bohrung (94) aufweist, die im Durchmesser dem Durchmesser des Führungsdrahts entspricht, und die Bohrung des Führungsteils im wesentlichen zur Längsachse des Gehäuses ausgerichtet ist, und das konische Ende des Führungsteils zur unmittelbaren Nachbarschaft der deformierbaren elastomeren Abdichtung verläuft, um das distale Ende eines Führungsdrahts gegen und durch den mindestens einen Öffnungsspalt der deformierbaren elastomeren Abdichtung und in den Katheter zu führen.

9. Selbstschließende Ventilvorrichtung nach Anspruch 6, worin das Führungsteil ein Niederdrückteil (78) aufweist, das innerhalb des Gehäuses zwischen der deformierbaren elastomeren Abdichtung und dem zweiten Ende des Gehäuses beweglich gehalten wird, das Niederdrückteil eine dadurch verlaufende zentrale Bohrung (80) aufweist, um den Durchtritt eines Führungsdrahts (30) oder von Fluiden dadurch zu ermöglichen, die zentrale Bohrung des Niederdrückteils mit der Längsachse des Gehäuses konzentrisch ist, das Niederdrückteil zur Bewegung längs der Längsachse des Gehäuses gehalten wird, das Niederdrückteil ein erstes Ende aufweist, das in unmittelbarer Nachbarschaft der deformierbaren elastomeren Abdichtung vorgesehen ist, um die deformierbare elastomere Abdichtung zu kontaktieren und deformieren, wenn es dagegen gedrückt wird, und das Niederdrückteil außerdem ein zweites gegenüberliegendes Ende aufweist, das in unmittelbarer Nachbarschaft des zweiten Endes des Gehäuses vorgesehen ist, und eine nach innen sich verjüngende konische Oberfläche aufweist, damit das konische Ende einer Spritze, eines Absperrhahns oder dergleichen, das mit dem zweiten Ende des Gehäuses verbunden ist, daran anstößt, die Einführung des konischen Endes der Spritze, des Absperrhahns oder dergleichen verursacht, dass das Niederdrückteil gegen die deformierbare elastomere Abdichtung drückt, um die Abdichtung aufzubrechen, damit Fluide hindurchtreten können.

10. Selbstschließende Ventilvorrichtung nach Anspruch 9, worin die zentrale Bohrung (54) des Gehäuses (48) einen zur deformierbaren elastomeren Abdichtung (76) unmittelbar benachbarten Teil mit verringertem Durchmesser (84) aufweist, der zwischen der deformierbaren elastomeren Abdichtung und dem ersten Ende des Gehäuses vorgesehen ist, wobei der Teil mit verringertem Durchmesser eine Schulter ausbildet, auf der die deformierbare elastomere Abdichtung ruht, wenn sie deformiert wird, wobei die Schulter eine übermäßige Deformation der deformierten elastomeren Abdichtung verhindert.

## Revendications

1. Dispositif obturateur autoétanche(10) pour un cathéter angiographique (14), le cathéter comprenant une extrémité distale (22) destinée à être insérée dans un vaisseau sanguin ou lymphatique du corps et une extrémité proximale opposée (12), le cathéter comprenant une lumière (28) s'étendant entre les extrémités distale et proximale pour laisser passer des fluides à travers elle et pour recevoir un fil de guidage souple (30) utilisé pour positionner le cathéter dans le corps, l'extrémité proximale du cathéter comprenant un élément de raccord à dispositif de verrouillage Luer femelle (32) adapté pour recevoir un élément de raccord à dispositif de verrouillage Luer mâle complémentaire (16) d'une seringue (18), d'un robinet d'arrêt ou analogue, afin de réaliser une liaison étanche au fluide avec ces derniers, ledit dispositif obturateur autoétanche comprenant :
a) un logement (48) comportant un alésage central (54) s'étendant entre des première (50) et seconde (52) extrémités opposées dudit logement, le long d'un axe longitudinal dudit logement ;
b) ladite première extrémité (50) dudit logement (48) comprenant un élément de raccord à dispositif de verrouillage Luer mâle (66) destiné à former un accouplement étanche au fluide avec l'élément de raccord à dispositif de verrouillage Luer femelle (32) au niveau de l'extrémité proximale (12) du cathéter (14) ;
c) ladite seconde extrémité dudit logement comprenant un élément de raccord à dispositif de verrouillage Luer femelle (58) adapté pour recevoir un élément de raccord à dispositif de verrouillage Luer mâle complémentaire (16) d'une seringue (18), d'un robinet d'arrêt ou analogue, afin de réaliser une liaison étanche au fluide avec ces derniers, ledit élément de raccord à dispositif de verrouillage Luer femelle comprenant un alésage conique convergeant vers l'intérieur (64) pour recevoir un embout de forme conique (74) d'une seringue, d'un robinet d'arrêt ou analogue ;
d) un joint d'étanchéité en élastomère déformable (76) supporté à l'intérieur dudit logement et s'étendant à travers l'alésage central de ce dernier pour rendre étanche sélectivement l'alésage central dudit logement, ledit joint d'étanchéité en élastomère déformable comprenant au moins une fente sensiblement alignée avec l'axe longitudinal dudit logement pour permettre au fil de guidage souple de passer à travers lui, tout en étant en contact étanche avec le fil de guidage souple pour empêcher le sang à l'intérieur du cathéter de passer au-delà dudit joint d'étanchéité en élastomère déformable, le joint d'étanchéité en élastomère déformable étant ouvert lors de l'accouplement d'un élément de raccord à dispositif de verrouillage Luer mâle (16) avec ladite seconde extrémité dudit logement afin de permettre au sang ou à d'autres fluides de passer à travers ledit joint d'étanchéité en élastomère déformable et à travers l'alésage central dudit logement une fois qu'une seringue, un robinet d'arrêt ou analogue est relié à ladite seconde extrémité dudit logement ; et
e) un moyen de guidage disposé de manière générale à proximité du joint d'étanchéité en élastomère déformable et dans lequel est réalisé un alésage central (80/94), ledit alésage central étant sensiblement concentrique avec l'axe longitudinal dudit logement, ledit alésage central ayant un diamètre qui permet au fil de guidage souple (30) de passer à travers le cathéter en étant centré et raidi lorsqu'il passe à travers ledit joint d'étanchéité en élastomère déformable.

2. Dispositif obturateur autoétanche pour un cathéter angiographique selon la revendication 1, dans lequel ledit joint d'étanchéité en élastomère déformable (76) est positionné à l'intérieur dudit logement à une certaine distance de ladite seconde extrémité (52) dudit logement, pour qu'ainsi ladite distance corresponde à une longueur prédéterminée sur laquelle un embout conique (16) d'une seringue (18), d'un robinet d'arrêt ou analogue s'étend dans ledit élément de raccord à dispositif de verrouillage Luer femelle (58) de ladite seconde extrémité (52) dudit logement (48), pour obliger l'embout conique de la seringue, du robinet d'arrêt ou analogue à entrer directement en contact avec ledit joint d'étanchéité en élastomère déformable et à le déformer pour permettre le passage de fluides à travers lui.

3. Dispositif obturateur autoétanche pour un cathéter angiographique selon la revendication 2, dans lequel ledit moyen de guidage est un élément de guidage (90) destiné à faciliter le passage de l'extrémité distale du fil de guidage à travers ladite fente dudit joint d'étanchéité en élastomère déformable, ledit élément de guidage comportant un embout conique (92) reçu par ladite seconde extrémité dudit logement, ledit embout conique dudit élément de guidage comprenant un alésage (94) s'étendant à travers lui et dont le diamètre est proportionné au diamètre du fil de guidage, l'alésage dudit élément de guidage étant sensiblement aligné avec l'axe longitudinal dudit logement, et l'embout conique dudit élément de guidage s'étendant à proximité dudit joint d'étanchéité en élastomère déformable pour guider l'extrémité distale d'un fil de guidage vers et à travers ladite fente dudit joint d'étanchéité en élastomère déformable et dans le cathéter.

4. Dispositif obturateur autoétanche pour un cathéter angiographique selon la revendication 1, dans lequel ledit moyen de guidage comprend un élément abaisseur (78) supporté de façon mobile à l'intérieur dudit logement, entre ledit joint d'étanchéité en élastomère déformable et la seconde extrémité dudit logement, ledit élément abaisseur comportant un alésage central (80) s'étendant à travers lui pour permettre le passage d'un fil de guidage ou de fluides, l'alésage central dudit élément abaisseur étant concentrique avec l'axe longitudinal dudit logement, ledit élément abaisseur étant supporté pouvoir se déplacer le long de l'axe longitudinal dudit logement, ledit élément abaisseur comportant une première extrémité disposée à proximité dudit joint d'étanchéité en élastomère déformable pour entrer en contact avec ce dernier et pour le déformer quand il est pressé contre lui, ledit élément abaisseur comprenant en outre une seconde extrémité opposée disposée à proximité de la seconde extrémité dudit logement et comportant une surface conique diminuant intérieurement pour être aboutée par l'embout conique d'une seringue, d'un robinet d'arrêt ou analogue relié à ladite seconde extrémité dudit logement, l'insertion de l'embout conique de la seringue, du robinet d'arrêt ou analogue obligeant ledit élément abaisseur à avancer vers ledit joint d'étanchéité en élastomère déformable pour le rompre afin de permettre à des fluides de passer à travers lui.

5. Dispositif obturateur autoétanche pour un cathéter angiographique selon la revendication 4, dans lequel l'alésage central (54) dudit logement (48) comprend une partie de diamètre réduit (84) à proximité dudit joint d'étanchéité en élastomère déformable, disposée entre ce dernier et la première extrémité dudit logement, ladite partie de diamètre réduit définissant un épaulement contre lequel ledit joint d'étanchéité en élastomère déformable s'appuie quand il est déformé, ledit épaulement empêchant une déformation excessive dudit joint d'étanchéité en élastomère déformable.

6. Dispositif obturateur autoétanche pour un cathéter angiographique selon la revendication 1, comprenant en outre :
(a) un cathéter angiographique (14) comprenant une extrémité distale (22) destinée à être insérée dans un vaisseau sanguin ou lymphatique du corps et une extrémité proximale opposée (12), ledit cathéter angiographique comprenant une lumière (28) s'étendant entre les extrémités distale et proximale pour laisser passer des fluides à travers elle et pour recevoir un fil de guidage (30) servant à le positionner dans le corps, l'extrémité proximale dudit cathéter comprenant un élément de raccord à dispositif de verrouillage Luer femelle (32) fixé audit un élément de raccord à dispositif de verrouillage Luer mâle (66) prévu au niveau de ladite première extrémité (50) dudit logement (48) afin de former un accouplement étanche au fluide avec ce dernier.

7. Dispositif obturateur autoétanche pour un cathéter angiographique selon la revendication 6, dans lequel l'embout conique (74) d'une seringue (18), d'un robinet d'arrêt ou analogue s'étend sur une longueur prédéterminée dans ledit élément de raccord à dispositif de verrouillage Luer femelle (58) de ladite seconde extrémité (52) dudit logement (48), et dans lequel ledit joint d'étanchéité en élastomère déformable (76) est positionné à l'intérieur dudit logement à une distance de ladite seconde extrémité dudit logement égale à environ ladite longueur prédéterminée pour obliger l'embout conique de la seringue, du robinet d'arrêt ou analogue à entrer directement en contact avec ledit joint d'étanchéité en élastomère déformable et à déformer ce dernier pour permettre le passage de fluides à travers lui.

8. Dispositif obturateur autoétanche selon la revendication 7, dans lequel ledit moyen de guidage est un élément de guidage (90) destiné à faciliter le passage de l'extrémité distale du fil de guidage (30) à travers ladite fente dudit joint d'étanchéité en élastomère déformable, ledit élément de guidage comportant un embout conique (92) reçu par ladite seconde extrémité (52) dudit logement, ledit embout conique dudit élément de guidage comprenant un alésage (94) s'étendant à travers lui et dont le diamètre est proportionné au diamètre du fil de guidage, l'alésage dudit élément de guidage étant sensiblement aligné avec l'axe longitudinal dudit logement, et l'embout conique dudit élément de guidage s'étendant à proximité dudit joint d'étanchéité en élastomère déformable pour guider l'extrémité distale d'un fil de guidage vers et à travers ladite fente dudit joint d'étanchéité en élastomère déformable et dans ledit cathéter.

9. Dispositif obturateur autoétanche selon la revendication 6, dans lequel ledit moyen de guidage comprend un élément abaisseur (78) supporté de façon mobile à l'intérieur dudit logement, entre ledit joint d'étanchéité en élastomère déformable et la seconde extrémité dudit logement, ledit élément abaisseur comportant un alésage central (80) s'étendant à travers lui pour permettre le passage d'un fil de guidage (30) ou de fluides, l'alésage central dudit élément abaisseur étant concentrique avec l'axe longitudinal dudit logement, ledit élément abaisseur étant supporté en vue de son déplacement le long de l'axe longitudinal dudit logement, ledit élément abaisseur comportant une première extrémité disposée à proximité dudit joint d'étanchéité en élastomère déformable pour entrer en contact avec ce dernier et le déformer quand il est pressé contre lui, ledit élément abaisseur comprenant en outre une seconde extrémité opposée disposée à proximité de la seconde extrémité dudit logement et comportant une surface conique diminuant intérieurement pour être aboutée par l'embout conique d'une seringue, d'un robinet d'arrêt ou analogue relié à ladite seconde extrémité dudit logement, l'insertion de l'embout conique de la seringue, du robinet d'arrêt ou analogue obligeant ledit élément abaisseur à avancer vers ledit joint d'étanchéité en élastomère déformable pour le rompre afin de permettre à des fluides de passer à travers lui.

10. Dispositif obturateur autoétanche selon la revendication 9, dans lequel l'alésage central (54) dudit logement (48) comprend une partie de diamètre réduit (84) à proximité dudit joint d'étanchéité en élastomère déformable (76), disposée entre ce dernier et la première extrémité dudit logement, ladite partie de diamètre réduit définissant un épaulement contre lequel ledit joint d'étanchéité en élastomère déformable s'appuie quand il est déformé, ledit épaulement empêchant une déformation excessive dudit joint d'étanchéité en élastomère déformable.
